# EUROPEAN PATENT SPECIFICATION

(11) **EP 2 513 258 B1**
(45) Date of publication and mention of the grant of the patent: **23.03.2016**
(21) Application number: 10841490.5
(22) Date of filing: 13.12.2010
(51) Int. Cl.: C10G 17/09, C10G 29/06, C10C 3/02

(54) **IMPROVED METHOD OF REMOVING HYDROGEN SULPHIDE**
VERBESSERTES VERFAHREN ZUR BESEITIGUNG VON WASSERSTOFFSULFID
PROCÉDÉ PERFECTIONNÉ D'ÉLIMINATION DE SULFURE D'HYDROGÈNE

(30) Priority: 15.12.2009 US 638365
(43) Date of publication of application: 24.10.2012
(60) Divisional application: 15175952.9
(73) Proprietor: Nalco Company, Naperville, IL 60563-1198 (US)
(72) Inventor: COMPTON, Dennis R., Sugar Land Texas 77479 (US); JEFFERIES, Samuel, Cornwall TR8 5SL (GB); SHARPE, Ron, Hampshire SO418FU (GB)
(74) Representative: Chalk, Anthony John
(86) International application number: PCT/US2010/060000
(87) International publication number: WO 2011/081860

(56) References cited:
- EP-A1- 0 121 377
- EP-A1- 0 421 683
- US-A- 5 000 835
- US-A- 5 000 835
- US-B1- 6 599 472
- ANDOR J A ET AL: "Physical and chemical modification of zinc carboxylate-containing lubricants by molecular structure changes", LUBRICATION SCIENCE, LEAF COPPIN PUBLISHING LTD, US, vol. 11, no. 2, 1 January 1999 (1999-01-01), pages 115-134, XP009128989, ISSN: 0954-0075
- ANDOR, J.A. LUBRICATION SCI. vol. 11, no. 2, 1999, pages 115 - 134, XP009128989

## Description

### Background of the Invention

This invention relates to the use of zinc octoates as hydrogen sulfide scavengers. Petroleum asphalt is produced as a residue of a thermal separation refinery process. The thermal separation process causes thermal cracking to occur which frequently causes hydrogen sulfide to be present in the asphalt stream. In fact, thermal cracking continues in the asphalt even after the asphalt has left the vacuum distillation section of the operation, particularly at high temperature. In order to permit the safe loading, handling, and storage of the asphalt, it is necessary to reduce the hydrogen sulfide to safe levels in the asphalt. This has been done in the past by weathering of the hot asphalt for sufficient time for the hydrogen sulfide to be reduced to safe levels. This not only takes a considerable amount of time (several days), but it releases hydrogen sulfide to the vapor space in the storage, which could create hazardous conditions. Moreover, recent emphasis on environmental regulations in Europe stresses the limits on the hydrogen sulfide content of vent gas.

Other efforts to avoid these problems involve operating the vacuum distillation tower at a lower temperature to reduce thermal cracking in the residue. Lower temperature operation is achieved by increasing the flow of asphalt in a quench loop. This, however, is less efficient than operating at higher temperatures and decreases throughput and thermal recovery. Other related methods of addressing this situation are described in European Patent Specification, Publication No. 0121377 and European Patent 000 421 683 A1.

US Patent 5,000,835 describes using metal carboxylates as hydrogen scavengers. This patent describes the reaction between metal carboxylates with 6 to 24 carbon atoms. In these metal carboxylates, the carbonyl group functions as a carrier for the oppositely charged metal and places the metal into a form which is soluble in an organic environment and able to make contact with dissolved hydrogen sulfide. When the metal in the metal carboxylates reacts with dissolved hydrogen sulfide, the two form insoluble metal sulfides, which eliminate the toxic and corrosive properties of the hydrogen sulfide. While this patent does mention the use of zinc octoate, which is oil soluble and readily available it also notes that zinc octoate is less effective than other metal carboxylates.

Thus there is clear need and utility for an improved method of using zinc octoate as a hydrogen sulfide scavenger. The art described in this section is not intended to constitute an admission that any patent, publication or other information referred to herein is "prior art" with respect to this invention, unless specifically designated as such. In addition, this section should not be construed to mean that a search has been made or that no other pertinent information as defined in 37 C.F.R. § 1.56(a) exists.

### Brief Summary of the Invention

According to the present invention there is provided a method for removing sulfides from petroleum streams, the method comprising the step of adding to the organic liquid an effective amount of a zinc octoate with a molar ratio of zinc to octanoic acid that is 2:3;
wherein the zinc octoate is tetranuclear oxo zinc octoate;
the zinc octoate is prepared in an aromatic solvent in which the zinc metal content comprises between 5% and 20% by weight of the fluid and wherein at least 50% of the sulfides are removed.

The invention is directed towards a method for removing sulfides from viscous petroleum streams such as asphalt, crude oil, and oil slurry. The zinc octoate is an oxo zinc octoate and it is tetranuclear oxo zinc octoate. The zinc octoate may be added in a fluid with a viscosity less than that of a similar fluid with a 1 :2 molar ratio of zinc to octanoic acid.

The dosage of zinc octoate added to the organic liquid may be from 1 to 2000 ppm. The addition reduces at least 50% of the sulfides. The zinc octoate is added in a low viscosity fluid in which zinc metal content comprises between 5% and 20% by weight of the fluid.

### Brief Description of the Drawings

A detailed description of the invention is hereafter described with specific reference being made to the drawings in which:

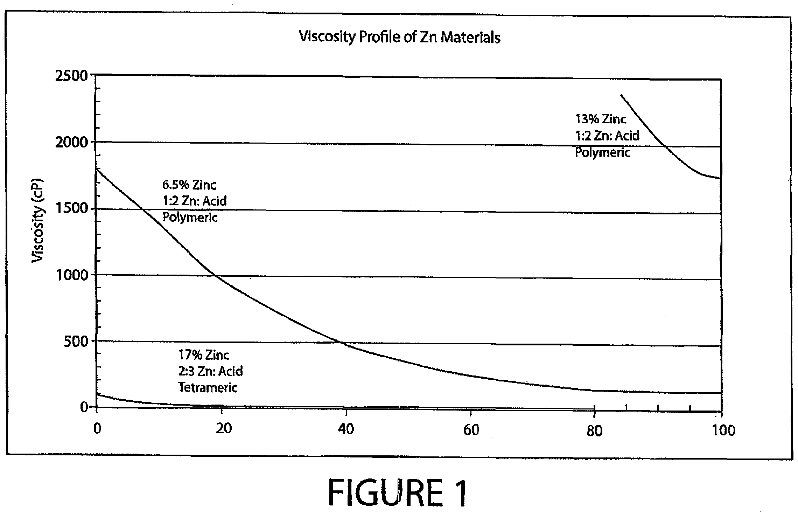

FIG. 1 is a graph showing the viscosity of various zinc octoates as hydrogen sulfide scavengers. The lower the viscosity of the material the easier it is to add and disperse into the asphalt

### Detailed Description of the Invention

Zinc octoate is an 8 -carbon carboxylic acid (specifically 2 ethyl hexanoic acid) in which a zinc ion coordinates with the oxygen atoms of the acid. Because zinc has a +2 charge and carboxylic acid has a -1 charge, it was previously assumed that all zinc octoates must have a 1 :2 ratio of zinc moieties to carboxylic acid. As represented in FIG. 1, these 1 :2 zinc octoates, tend to polymerize and form highly viscous materials, which makes their practical usefulness as a hydrogen sulfide scavenger very limited.

The oxo-zinc group is a tetranulcear oxo-zinc in which four zinc atoms are bonded with one oxygen group and forms a 2:3 ratio of zinc to carboxylic acid.

This ratio facilitates the reaction of all of the zinc and prevents the appearance of haze, which is indicative of residual unreacted zinc oxide.

The use of the ratio of zinc octoates describes herein imparts a number of advantages. Firstly the resulting octoate has more zinc atoms to be present per mol of octoate. Because the zinc atoms are the primary impetus of removing the sulfides, concentrating more zinc per mol increases the effectiveness of the octoate. Secondly as shown in FIG. 1, non 1:2 ratio zinc octoates have lower viscosities than 1:2 octoates resulting in an octoate which is more applicable and which can have a concentration that is more effective than more viscous zinc octoates. The lower viscosity is quite an unexpected result as one would think that a complex that binds more molecules together would have a higher viscosity, yet the test results demonstrate that when in this 2:3 ratio a lower viscosity results. FIG. 1 illustrates this reduced viscosity relative to temperature for the tetra oxo and polymer forms of zinc octoate prepared in an aromatic solvent.

The chemical structure of tetranuclear oxo zinc octoate is:

µ-Zn4O-µ -(O2C8H15)6

### EXAMPLES

The foregoing may be better understood by reference to the following example, which is presented for purposes of illustration and is not intended to limit the scope of the invention.

A number of samples were prepared in the same aromatic solvent. The various samples were tested for vapor space H₂S levels using Draeger Tubes. Table I illustrates the sample's effectiveness after heating for 2 hours at temperatures of 157-163°C (315 - 325 °F) Table II illustrates that the inventive composition is highly effective even after shorter time periods.

**TABLE I**

| | | | **H2S Level (ppm)** | |
|---|---|---|---|---|
| **Test No.** | **Additive Description** | **Additive Treat in Asphalt (ppm)** | **No Treatment** | **With Treatment** |
| 1 | 5.5% Iron Octoate | 99 | 4500 | 3250 |
| 2 | 5.5% Iron Octoate | 301 | 4500 | 2700 |
| 3 | 5.5% Iron Octoate | 702 | 3500 | 425 |
| 4 | 5.5% Iron Octoate | 1769 | 3500 | 40 |
| | 17% Zinc Octoate | | | |
| 5 | tetrameric complex | 100 | 4500 | 2250 |
| | 17% Zinc Octoate | | | |
| 6 | tetrameric complex | 291 | 4500 | 1100 |
| | 17% Zinc Octoate | | | |
| 7 | tetrameric complex | 541 | 3500 | 75 |
| | 17% Zinc Octoate | | | |
| 8 | tetrameric complex | 695 | 3500 | 30 |
| | 17% Zinc Octoate | | | |
| 9 | tetrameric complex | 1744 | 3500 | 0 |
| | 17% Zinc Octoate | | | |
| 10 | tetrameric complex | 988 | 5000 | 10 |
| | 13% Zinc Octoate | | | |
| 11 | polymeric complex | 996 | 5000 | 30 |
| | 6.5% Zinc Octoate | | | |
| 12 | polymeric complex | 979 | 5000 | 775 |

**TABLE II**

| | | | | **H2S Level ( ppm)** | |
|---|---|---|---|---|---|
| **Test No.** | **Additive Description** | **Additive Treat in Asphalt (ppm)** | **Time** | **No Treatment** | **With Treatment** |
| | 17% Zinc Octoate | | | | |
| 1 | tetrameric complex | 1013 | 1 min | 5000 | 100 |
| | 17% Zinc Octoate | | | | |
| 2 | tetrameric complex | 998 | 30 min | 5000 | 35 |
| | 17% Zinc Octoate | | | | |
| 3 | tetrameric complex | 991 | 2 hours | 5000 | 20 |
| | 17% Zinc Octoate | | | | |
| 4 | tetrameric complex | 994 | 4 hours | 5000 | 5 |

## Claims

1. A method for removing sulfides from petroleum streams, the method comprising the step of adding to the organic liquid an effective amount of a zinc octoate with a molar ratio of zinc to octanoic acid that is 2:3;
wherein the zinc octoate is tetranuclear oxo zinc octoate;
the zinc octoate is prepared in an aromatic solvent in which the zinc metal content comprises between 5% and 20% by weight of the fluid and wherein at least 50% of the sulfides are removed.

2. The method of claim 1 wherein the petroleum stream is one selected from the list consisting of; asphalt, crude oil, oil slurry, and any combination thereof.

## Patentansprüche

1. Verfahren zum Entfernen von Sulfiden aus Mineralölströmen, wobei das Verfahren den Schritt des Hinzufügens einer wirksamen Menge eines Zinkoktoats mit einem molaren Verhältnis Zink zu Octansäure von 2:3 zu der organischen Flüssigkeit umfasst;
wobei das Zinkoktoat tetranukleares Oxo-Zinkoktoat ist;
das Zinkoktoat in einem aromatischen Lösungsmittel bereitgestellt wird, in welchem der Zinkmetallgehalt zwischen 5 Gew.-% und 20 Gew.-% der Flüssigkeit beträgt und wobei mindestens 50 % der Sulfide entfernt werden.

2. Verfahren nach Anspruch 1, wobei der Mineralölstrom ein aus einer Liste ausgewählter ist, die sich aus Asphalt, Rohöl, Ölschlamm und beliebigen Kombinationen daraus zusammensetzt.

## Revendications

1. Procédé d'extraction de sulfures à partir de courants de pétrole, le procédé comprenant l'étape d'addition au liquide organique une quantité adéquate d'un octoate de zinc avec un rapport molaire de zinc à l'acide octanoïque qui est de 2:3;
où l'octoate de zinc est un octoate d'oxozinc tétranucléaire;
l'octoate de zinc est préparé dans un solvant aromatique dans lequel la teneur en métal de zinc comprend entre 5% et 20% en poids du fluide et où au moins 50% des sulfures sont retirés.

2. Procédé selon la revendication 1, où le courant de pétrole est sélectionné dans la liste composée d'asphalte, de pétrole brut, de boue de pétrole et de toute combinaison de ceux-ci.
